(19) **Europäisches Patentamt / European Patent Office / Office européen des brevets**

(11) **EP 3 330 710 B1**

(12) **EUROPEAN PATENT SPECIFICATION**

(45) Date of publication and mention
of the grant of the patent:
**09.09.2020 Bulletin 2020/37**

(21) Application number: **16832913.4**

(22) Date of filing: **28.07.2016**

(51) Int Cl.:
*G01N 33/574* (2006.01)     *C12Q 1/6886* (2018.01)
*A61K 39/395* (2006.01)     *A61K 48/00* (2006.01)
*C07K 16/30* (2006.01)     *A61P 35/00* (2006.01)

(86) International application number:
**PCT/JP2016/072204**

(87) International publication number:
**WO 2017/022634 (09.02.2017 Gazette 2017/06)**

(54) **METHOD AND KIT FOR EVALUATING PROGNOSIS, DISTANT RECURRENCE RISK AND INVASION OF GLIOMA, AND PHARMACEUTICAL COMPOSITION FOR TREATING GLIOMA**

VERFAHREN UND KIT ZUR BEURTEILUNG DER PROGNOSE, DES ENTFERNTEN REZIDIVRISIKOS UND DER INVASION VON GLIOMEN SOWIE PHARMAZEUTISCHE ZUSAMMENSETZUNG ZUR BEHANDLUNG VON GLIOMEN

PROCÉDÉ ET KIT PERMETTANT D'ÉVALUER UN PRONOSTIC, UN RISQUE DE RÉCIDIVE À DISTANCE ET UNE INVASION DE GLIOMES AINSI QUE COMPOSITION PHARMACEUTIQUE POUR TRAITER UN GLIOME

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.07.2015 JP 2015151838**

(43) Date of publication of application:
**06.06.2018 Bulletin 2018/23**

(73) Proprietor: **Sapporo Medical University**
**Sapporo-shi, Hokkaido 060-8556 (JP)**

(72) Inventors:
• **HONMOU Osamu**
**Sapporo-shi**
**Hokkaido 060-8556 (JP)**
• **WANIBUCHI Masahiko**
**Sapporo-shi**
**Hokkaido 060-8556 (JP)**
• **OHTAKI Shunya**
**Sapporo-shi**
**Hokkaido 060-8556 (JP)**
• **SASAKI Masanori**
**Sapporo-shi**
**Hokkaido 060-8556 (JP)**
• **SASAKI Yuko**
**Sapporo-shi**
**Hokkaido 060-8556 (JP)**
• **MIKUNI Nobuhiro**
**Sapporo-shi**
**Hokkaido 060-8556 (JP)**

(74) Representative: **Wasner, Marita**
**Niizuma Wasner GmbH**
**Patentanwaltskanzlei**
**Postfach 278**
**4125 Riehen 1 (CH)**

(56) References cited:
**WO-A1-2010/064702     WO-A2-2008/054786**
**JP-A- 2011 528 804     JP-A- 2013 500 018**
**JP-A- 2013 512 000     JP-A- 2013 526 852**

• **AMANDA FARAGE FRADE ET AL: "Polymorphism in the Alpha Cardiac Muscle Actin 1 Gene Is Associated to Susceptibility to Chronic Inflammatory Cardiomyopathy", PLOS ONE, vol. 8, no. 12, 19 December 2013 (2013-12-19), page e83446, XP055548261, DOI: 10.1371/journal.pone.0083446**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

- Shunya Ohtaki ET AL: "ACTC1 as an invasion and prognosis marker in glioma", Journal of Neurosurgery, Vol. 126, N.2, 1 February 2017 (2017-02-01), pages 467-475, XP055513502, DOI: 10.3171/2016.1.JNS152075 Retrieved from the Internet: URL:https://thejns.org/view/journals/j-neu rosurg/126/2/article-p467.xml [retrieved on 2018-10-09]
- WANIBUCHI MASAHIKO ET AL: "Actin, alpha, cardiac muscle 1 (ACTC1) knockdown inhibits the migration of glioblastoma cellsin vitro", JOURNAL OF NEUROLOGICAL SCIENCES, ELSEVIER SCIENTIFIC PUBLISHING CO, AMSTERDAM, NL, vol. 392, 17 July 2018 (2018-07-17), pages 117-121, XP085438181, ISSN: 0022-510X, DOI: 10.1016/J.JNS.2018.07.013

- VIGNESWARAN K ET AL.: 'Beyond the World Health Organization grading of infiltratinggliomas: advances in the molecular genetics of glioma classification' ANN TRANSL MED. vol. 3, no. 7, 2015, page 95, XP055363125
- OHTAKI S ET AL.: 'ACTC1 as an invasion and prognosis marker in glioma' J NEUROSURG. 15 April 2016, pages 1 - 9, XP055513502

**Description**

Technical Field

The present invention relates to a method for determining the prognosis of a glioma, the risk of remote recurrence of a glioma, the invasion ability of a glioma cell, or the presence of a highly invasive glioma in a sample, the use of a kit in the determination method, and a pharmaceutical composition for treating a glioma.

Background Art

A glioma is a malignant tumor that occurs in the brain, and is a major class of brain tumors. Gliomas are classified into four grades (Grade I to VI) according to criteria of the World Health Organization (WHO). Grade III and Grade IV gliomas are called high grade gliomas, and have high proliferating ability and invasion ability. Among high grade gliomas, glioblastomas classified into Grade IV (GBM) have the highest malignancy, and the five-year survival rate thereof is only about 10%.

A glioma is a disease that is difficult to be completely removed by surgery because tumor cells invade as if they infiltrated into the brain and the boundary between the glioma and the normal tissue is unclear, and because the tumor is difficult to be removed depending on the location where the tumor occurs. Therefore, a standard therapy of gliomas, especially high grade gliomas and glioblastomas is to surgically remove tumors as much as possible, followed by radiotherapy and chemotherapy with temozolomide to prevent or retard the recurrence. However, even when such therapy is conducted properly, the high invasion ability of the glioblastoma is liable to develop recurrence frequently in a remote area distanced from the original location, and this is one cause of poor prognosis of glioblastoma. Therefore, the information regarding whether the glioma is a high grade glioma, or further is a glioblastoma, and the information regarding whether or not the glioma easily develops into a high grade glioma, or further into a glioblastoma is useful for the formulation of a therapeutic strategy for the glioma patient.

As a factor related to the prognosis of a glioma, many genetic factors such as PTEN, p16$^{INK4a}$ deletion, MDM2, EGFR, and TP53 have been reported. Among these, IDH mutation is considered as a favorable prognostic factor for high grade gliomas, and methylation of MGMT promoter is considered as a predictive factor for temozolomide response. Other than these, further studies on glioma markers are being developed (for example, Non-Patent Literature 1). WO2008/054786 discloses the use of glycogene synthase kinase 3 as marker for evaluating invasion or migration of glial cells and as therapeutic target for treating gliomas,

Citation List

Non-Patent Literatures

Non-Patent Literature 1: Vigneswaran K et al., Ann. Transl. Med. 2015; 3 (7): 95.

Summary of Invention

Technical Problem

It is an object of the present invention to provide a novel biomarker capable of informing the prognosis of a glioma, the risk of remote recurrence of a glioma, the invasion ability of a glioma cell and the like at the time of detection or in the early stage of therapy, or capable of being used for on-site diagnosis at the time of surgical removal of the glioma.

Solution to the Problem

The inventors proceeded with the study focusing on actin, alpha cardiac muscle 1 (ACTC1) which is one of actin families involved in cell motility, and found that a glioma in which expression of mRNA encoding ACTC1 protein was detected showed poorer prognosis compared with a glioma in which expression of mRNA encoding ACTC1 protein was not detected, and accomplished the following aspects of invention.

(1) A method for determining the prognosis of a glioma, including: a step of detecting ACTC1 protein and/or mRNA encoding ACTC1 protein in a sample containing a glioma collected from a patient, and a step of determining that the glioma has a poor prognosis when ACTC1 protein and/or mRNA encoding ACTC1 protein is detected in the detecting step.

(2) A method for determining the risk of remote recurrence of a glioma, including: a step of detecting ACTC1 protein and/or mRNA encoding ACTC1 protein in a sample containing a glioma collected from a patient, and a step of determining that the glioma has a high risk of remote recurrence when ACTC1 protein and/or mRNA encoding ACTC1 protein is detected in the detecting step.

(3) A method for determining the invasion ability of a glioma cell, including: a step of detecting ACTC1 protein and/or mRNA encoding ACTC1 protein in a sample containing a glioma cell, and a step of determining that the glioma cell has a high invasion ability when ACTC1 protein and/or mRNA encoding ACTC1 protein is detected in the detecting step.

(4) A method for determining the presence of a highly invasive glioma in a sample collected from a glioma lesion or the vicinity of the glioma lesion in a patient, including a step of detecting ACTC1 protein and/or mRNA encoding ACTC1 protein in the sample, and a step of determining that the sample contains a highly invasive glioma when ACTC1 protein and/or mRNA encoding ACTC1 protein is detected in the detecting step.

(5) Use of a kit in a method according to claim 1 or 2 for determining the prognosis of a glioma and/or the risk of remote recurrence of a glioma, including a means that detects ACTC1 protein and/or mRNA encoding ACTC1 protein in a sample containing a glioma collected from a patient.

(6) Use of a kit in a method according to claim 3 for determining the invasion ability of a glioma cell, including a means that detects ACTC1 protein and/or mRNA encoding ACTC1 protein in a sample containing a glioma cell.

(7) Use of a kit in a method according to claim 4 for determining the presence of a highly invasive glioma in a sample collected from a glioma lesion or the vicinity of the glioma lesion in a patient, including a means that detects ACTC1 protein and/or mRNA encoding ACTC1 protein in the sample.

(8) A kit according to any one of (5) to (7), including at least one of a specific antibody for detecting ACTC1 protein or a primer nucleic acid or a probe nucleic acid for detecting mRNA encoding ACTC1.

(9) A pharmaceutical composition comprising a substance that suppresses the expression and/or function of ACTC1 protein for use in treating glioma , wherein the substance that suppresses the expression and/or function of ACTC1 protein is selected from the group consisting of an inhibitory nucleic acid against a gene encoding ACTC1 protein, a recombinant virus capable of expressing an inhibitory nucleic acid against a gene encoding ACTC1 protein, and a neutralizing antibody against ACTC1 protein.

Advantageous Effects of the Invention

According to the present invention, it is possible to determine the prognosis of a glioma, the risk of remote recurrence of a glioma, the invasion ability of a glioma cell and the like by using a novel biomarker, ACTC1 protein and/or mRNA encoding ACTC1 protein. Also in a craniotomy, by examining the expression of the biomarker according to the present invention in a visible lesion or the vicinity of the lesion, it becomes possible to excise an appropriate area without leaving the site that is suspected to be the highly invasive glioma. Further, ACTC1 protein is a therapeutic target for a glioma, in particular, a high grade glioma, and a substance that suppresses the expression and/or function of ACTC1 protein can be used as a pharmaceutical for treating a glioma.

Brief Description of the Drawings

Fig. 1 illustrates graphs showing the expression rate (Fig. 1A) and the expression level (Fig. 1B) of ACTC1 in glioma tissues for each WHO-defined grade. In Fig. 1B, the error bars indicate the standard errors of the means, and the vertical axis indicates the relative expression level (fold change, FC).

Fig. 2 illustrates scatter plots showing the relationship between the ACTC1-mRNA relative expression level (fold change, FC) in glioma tissues and the overall survival. The upper section covers all the gliomas excluding Grade I, and the lower section covers gliomas of Grade IV.

Fig. 3 illustrates graphs showing Kaplan-Meier curves of the overall survival (Figs. 3A and 3C) and the progression free survival (Figs. 3B and 3D) for ACTC1-positive or ACTC1-negative glioma patients. A and B in the upper section cover all the gliomas excluding Grade I, and C and D in the lower section cover gliomas of Grade IV. In the graphs, the solid line indicates a ACTC1-positive group, and the broken line indicates a ACTC1-negative group.

Fig. 4 illustrates contrast-enhanced T1-weighted images showing typical MRI findings at the time of diagnosis of a ACTC1-positive glioblastoma (Figs. 4A to 4C) and a ACTC1-negative glioblastoma (Figs. 4D to 4F).

Fig. 5 illustrates images showing typical MRI findings at the time of recurrence of a ACTC1-positive glioblastoma (Figs. 5A to 5D) and a ACTC1-negative glioblastoma (Figs. 5E to 5H). A, C, E, and G in the upper section are FLAIR images, and B, D, F, and H in the lower section are contrast-enhanced T1-weighted images.

Fig. 6 illustrates scatter plots showing the relations between the ACTC1-mRNA relative expression level (fold change, FC) and patient age (Fig. 6A), Karnofsky performance status (KPS) (Fig. 6B) and MIB-1 index (Fig. 6C).

Fig. 7 illustrates immunostained photographs indicating the expression of ACTC1 protein in glioblastoma tissues. The left picture shows stained nuclei, the middle picture shows stained ACTC1, and the right picture is the merge of them.

Fig. 8 illustrates immunostained photographs indicating the expression of ACTC1 protein in glioblastoma cell strain U87. The left picture shows stained nuclei, the middle picture shows stained ACTC1, and the right picture is the merge of them.

Fig. 9 illustrates graphs showing the expression level of ACTC1-mRNA in siRNA-treated glioblastoma cell strain U87 (Fig. 9A), and the migration ability of said cell (Fig. 9B).

Description of the Embodiments

A method which is a first aspect of the present invention relates to a method for determining the prognosis of a glioma or the risk of remote recurrence of a glioma, the invasion ability of a glioma cell or the presence of a highly invasive glioma in a sample collected from a glioma lesion or the vicinity of the glioma lesion, and each method includes a step of detecting ACTC1 protein and/or mRNA encoding ACTC1 protein in a sample containing a glioma collected from a patient, a sample containing a glioma cell, or a sample collected from a glioma lesion or the vicinity of the glioma lesion in a patient.

Hereinafter, when ACTC1 protein and mRNA encoding ACTC1 protein are indicated in the meaning of a biomarker which is a target for detection in the present invention, they are denoted by "the present biomarker", whereas when ACTC1 protein and mRNA encoding ACTC1 protein are indicated in the meaning of protein and mRNA, they are denoted by "ACTC1 protein" and "ACTC1-mRNA", respectively.

ACTC1 protein is an isoform protein of $\alpha$ actin expressed in the skeletal muscles, and is known to be expressed in the cardiac sarcomeres, and involved in muscle contraction in heart beating. An amino acid sequence of human ACTC1 and a base sequence of cDNA encoding the same are registered in GenBank under the accession numbers AAH09978 and BC009978, respectively. While ACTC1 proteins have been studied focusing on expression and function in the myocardium, the expression of ACTC1 in the brain and, in particular, the relationship between malignancy of a glioma and the expression of ACTC1 protein have not been known.

The first aspect of the present invention uses ACTC1 protein and/or ACTC1-mRNA as a biomarker for determining each of the prognosis of a glioma or the risk of remote recurrence of a glioma, the invasion ability of a glioma cell or the presence of a highly invasive glioma in a sample collected from a glioma lesion or the vicinity of the glioma lesion. Specifically, the presence or absence of expression of the present biomarker in a sample containing a glioma collected from a patient, a sample containing a glioma cell, or a sample collected from a glioma lesion or the vicinity of the glioma lesion in a patient is used for an index for determining the prognosis of the glioma or the risk of remote recurrence of the glioma, the invasion ability of the glioma cell or the presence of a highly invasive glioma in the sample collected from the glioma lesion or the vicinity of the glioma lesion, respectively.

The amino acid sequence of ACTC1 protein and the base sequence of ACTC1-mRNA which are targets for detection in the present invention are not limited to the amino acid sequence and the base sequence registered under the accession numbers AAH09978 and BC009978, and mRNAs with a base sequence having, for example, a single base substitution (Single Nucleotide Polymorphism: SNP) and ACTC1 proteins with an amino acid sequence having substitution of an amino acid residue that can arise by such a base substitution are also included as targets for detection.

Detection of the present biomarker is carried out using a sample containing a glioma collected from a patient, a sample containing a glioma cell or a sample collected from a glioma lesion or the vicinity of the glioma lesion collected from a patient. These samples may be used for detection in the conditions as they are collected from a patient, or in the condition of cells as they are, or may be used after undergoing a general treatment for the purpose of detection of protein or mRNA, or may be used after undergoing a treatment by a general storage method such as formalin fixation.

In the detection of the present biomarker according to the present invention, either one or both of ACTC1 protein and ACTC1-mRNA may be detected.

The present biomarker in a sample can be detected by known methods. For example, in the case of ACTC1 protein, ACTCT1 protein may be detected by a known method such as an ELISA (enzyme-linked immunosorbent assay) method including a direct competitive method, an indirect competitive method, and a sandwich method, an RIA (radioimmunoassay) method, in situ hybridization, an immunoblotting analysis, a Western blotting analysis, and a tissue array analysis using a specific antibody therefor. In this case, the specific antibody is not limited by the animal species from which it is derived, and may be either a polyclonal antibody or a monoclonal antibody, and may be an antibody composed of the overall length of immunoglobulin or a partial fragment such as a Fab fragment or a F(ab')2 fragment.

A specific antibody against ACTC1 protein may be labeled with a fluorescent substance (e.g., FITC, rhodamine, phalloidin, etc.), colloidal particles such as gold, fluorescent micro beads such as Luminex (registered trademark, available from Luminex), heavy metal (e.g., gold, platinum, etc.), a pigment protein (e.g., phycoerythrin, phycocyanin, etc.), a radioactive isotope (e.g., $^3$H, $^{14}$C, $^{32}$P, $^{35}$S, $^{125}$I, $^{131}$I, etc.), an enzyme or the like (e.g., peroxidase, alkaline phosphatase, etc.), biotin, streptavidin or another labeling compound.

Detection of ACTC1-mRNA can be conducted by a known method capable of detecting expression of mRNA, such as a PCR method using a primer nucleic acid having an appropriate base sequence designed on the basis of the base sequence of ACTC1-mRNA, a hybridization method using a probe nucleic acid having a base sequence capable of hybridizing with the base sequence of ACTC1-mRNA under a stringent condition, or a microarray method using a chip to which a nucleic acid having a base sequence capable of hybridizing with the base sequence of ACTC1-mRNA is immobilized. The nucleic acid may be labeled with a fluorescent substance, a radioactive isotope, an enzyme, biotin, streptavidin or another labeling compound depending on the method to be used.

The method which is the first aspect of the present invention relates to a method for determining the prognosis of a glioma or the risk of remote recurrence of a glioma, the invasion ability of a glioma cell or the presence of a highly invasive glioma in a sample collected from a glioma lesion or the vicinity of the glioma lesion, and includes the step of determining that the prognosis of the glioma is poor, or the risk of remote recurrence is high, the invasion ability of the glioma cell is high, or highly invasive gliomas are present in the sample when the present biomarker is detected in the sample. The method which is the first aspect of the present invention can be represented by a method for collecting or providing data regarding the expression of ACTC1 protein and/or mRNA encoding ACTC1 protein in a sample for determining or for being subjected to determination of the prognosis of a glioma or the risk of remote recurrence of a glioma, the invasion ability of a glioma cell or the presence of a highly invasive glioma in a sample collected from a glioma lesion or the vicinity of the glioma lesion.

As will be shown in the later Examples, the presence or absence of expression of the present biomarker in pathological tissues of a total of 50 glioma patients diagnosed as WHO Grade I to IV was investigated, and the correlations between the survival after treatment, the occurrence of remote recurrence, and the records regarding the degree of invasion by MRI findings at the time of diagnosis were analyzed for each patient. As a result, it was confirmed that the expression of the present biomarker shows statistically significant positive correlations with short survival, remote recurrence, and a high degree of invasion.

This enables a physician to know the malignancy of the glioma and predict the influence of the same by examining the presence or absence of expression of the present biomarker in a glioma patient. For a patient in which the present biomarker is detected, a physician can formulate and perform a therapeutic strategy for the glioma patient on the assumption that the glioma is malignant. Also in a craniotomy for the purpose of the excision of the glioma, by examining the expression of the present biomarker in a visible lesion or the vicinity of the lesion, it is possible to know whether a highly invasive glioma exists in the lesion or in the vicinity of the lesion. Therefore, it becomes possible to conduct a so-called on-site diagnosis for determining that the lesion or the vicinity of the lesion is an area to be excised when a highly invasive glioma exists, and it is possible to reduce the risk of recurrence of the glioma after the surgical operation.

Also as shown in the later Examples, the expression rate of the present biomarker elevates as the WHO grade elevates, however, the expression rate is at most more than half even in Grade IV. Thus, it is considered that the present biomarker and the WHO grade are not closely correlated. Therefore, the method which is the first aspect of the present invention, targets not only gliomas of high grade WHO Grade III and IV glioma (high grade glioma), and glioblastomas, but also gliomas of all grades. In the present specification, the term "glioma", when used alone, covers gliomas of all grades, and high grade gliomas and glioblastomas.

For determination in the present invention, detecting presence or absence of expression of the present biomarker is enough. Regarding presence or absence of expression, when the expression of the present biomarker is not actually recognized with the detection sensitivity for each employed detection method or expression is not more than the detection limit, it can be determined that expression is not observed or negative, whereas when expression exceeds such a degree, it can be determined that expression is observed or positive.

The wording "expression of the present biomarker is not actually recognized" indicates that the present biomarker can be detected, but the quantified value of the biomarker is lower than a certain cutoff value. Such a cutoff value can be set, for example, on the basis of the expression level of ACTC1 in a sample in which lack of expression of ACTC1 is known.

Concretely, the expression level of ACTC1 in a reference sample in which lack of expression of ACTC1 is known, such as normal glia cells or a normal brain tissue that is not affected with a glioma, is measured, and the obtained value is set as a cutoff value. Then the ACTC1 expression level in the sample to be determined is measured in the same manner, and the obtained value is compared with the cutoff value. If the obtained value is less than the cutoff value, it can be determined that expression is not observed or negative, whereas if the obtained value is not less than the cutoff value, it can be determined that expression is observed or positive.

The expression of the present biomarker can be quantified by a comparative Ct method ($\Delta\Delta$Ct method, also called a cycle comparative method) in real-time PCR. Concretely, a calibrator mRNA derived from a sample in which lack of expression of ACTC1 is known, such as normal glia cells or a normal brain tissue that is not affected with a glioma, and a mRNA derived from a sample to be determined are subjected to real-time PCR respectively, and a corresponding Ct value is obtained from amplification curves of ACTC1 gene and an intrinsic control gene. Then, for each of the sample to be determined and the calibrator, $\Delta$Ct is calculated as the difference between the Ct value of ACTC1 gene and the Ct value of the intrinsic control gene. From $\Delta\Delta$Ct obtained as the difference between $\Delta$Ct of the sample to be determined and $\Delta$Ct of the calibrator, FC = $2^{-\Delta\Delta Ct}$ which is a relative expression level of ACTC1 gene is calculated. The calculated FC is compared with the cutoff value, and if FC is less than the cutoff value, it can be determined that the expression is not observed or negative, whereas if FC is not less than the cutoff value, it can be determined that the expression is observed or positive.

The cutoff value of FC is particularly preferably 1. FC = 1 means that the difference between $\Delta$Ct of the sample to be determined, and $\Delta$Ct of the calibrator is 0, or in other words, it means that there is no difference between the ACTC1-mRNA amount in the sample to be determined, and the ACTC1-mRNA amount in the calibrator.

Also as shown in the later Examples, since there is a certain correlation between the expression level of the present biomarker and the grade of glioma, the quantitative expression level of the present biomarker may be supplementarily used for the determination.

A second aspect of the present disclosure relates to a kit for determining the prognosis of a glioma and/or the risk of remote recurrence of a glioma, including a means that detects ACTC1 protein and/or mRNA encoding ACTC1 protein which is the present biomarker in a sample containing a glioma collected from a patient.

A third aspect of the present disclosure relates to a kit for determining the invasion ability of a glioma cell, including a means that detects ACTC1 protein and/or mRNA encoding ACTC1 protein which is the present biomarker in a sample containing a glioma cell. The kit of the present aspect can also be used for determining the invasion ability of established cell lines of gliomas, in addition to a glioma cell contained in a sample collected from a patient.

A fourth aspect of the present disclosure relates to a kit for determining the presence of a highly invasive glioma in a sample collected from a glioma lesion or the vicinity of the glioma lesion in a patient, including a means that detects ACTC1 protein and/or mRNA encoding ACTC1 protein which is the present biomarker in the sample. The kit of the present aspect is useful for the aforementioned on-site diagnosis.

Any kit of the second to the fourth aspects can be used in the first aspect. The kits of these aspects preferably contain at least one of substances usable for the detection of the present biomarker, for example, a specific antibody against ACTC1 protein, or a primer nucleic acid or a probe nucleic acid for detecting ACTC1-mRNA, and may contain a buffer, a coloring reagent, dNTP, and any other reagent that is used in conducting immunological reaction or PCR reaction and so on.

A fifth aspect of the present invention relates to a pharmaceutical composition for treating a glioma, including a substance that suppresses the expression and/or function of ACTC1 protein as defined in the claims.

Actin is known to be a molecule that is involved in the regulation of cell motility and shape, and is reported to be involved particularly in invasion ability or metastasis in cancer cells. Also regarding glioma, one of the causes of its invasion and remote recurrence is considered to be the enhancement of cell motility provided by cytoskeletal filaments that consist of three major components: actin filaments, microtubules, and intermediate filaments. Therefore, ACTC1 protein, of which expression has positive correlation with a poor prognosis, remote recurrence and a high invasion ability of a glioma, is highly possibly a molecule that can cause the poor prognosis, remote recurrence and the high invasion ability of a glioma, and a substance that suppresses the expression and/or function of ACTC1 protein and/or a composition containing the same are able to suppress the remote recurrence and invasion ability of a glioma, and are expected to be usable as a pharmaceutical or a pharmaceutical composition for treating a glioma.

Examples of the substance that suppresses the expression and/or function of ACTC1 protein include a neutralizing antibody against ACTC1 protein, a compound that binds with ACTC1 protein and inhibits the function of ACTC1 protein, and an inhibitory nucleic acid such as an siRNA, shRNA or micro RNA capable of inhibiting transcription or translation from a gene encoding ACTC1 protein.

The pharmaceutical composition according to the present invention may contain only one or a plurality of substances that suppress expression and/or function of ACTC1 protein, and may further contain other pharmaceutical components and/or any pharmaceutically acceptable excipients or other components.

While the pharmaceutical composition according to the present invention may be an oral preparation or a parenteral preparation, it is preferably used in the form of a parenteral preparation such as an injection or a drip. Examples of the carrier that can be used in a parenteral preparation include aqueous carriers that are ordinarily used in cell preparations, such as saline, or an isotonic solution containing glucose, D-sorbitol and so on.

The pharmaceutical composition according to the present invention may be encapsulated and/or immobilized in/to an appropriate DDS such as a polymeric micelle, liposome, emulsion, microsphere and a nanosphere.

Further, when the pharmaceutical composition according to the present invention contains an inhibitory nucleic acid, the inhibitory nucleic acid can be introduced into a glioma cell by using any known cell introducing technique such as a calcium phosphate method, a lipofection method, an ultrasonic introducing method, an electroporation method, a particle gun method, a method using a viral vector (e.g., herpes virus, adenovirus or retrovirus) or a micro injection method.

When a viral vector is used, namely when the pharmaceutical composition contains a recombinant virus capable of expressing an inhibitory nucleic acid for a gene encoding ACTC1 protein, the dosage ranges, for example, from $1 \times 10^3$ to $1 \times 10^{14}$, preferably from $1 \times 10^5$ to $1 \times 10^{12}$, more preferably from $1 \times 10^6$ to $1 \times 10^{11}$, most preferably from $1 \times 10^7$ to $1 \times 10^{10}$ plaque formation unit (p.f.u.) per one human subject.

While the administration method of the pharmaceutical composition according to the present invention is not particularly limited, in the case of a parenteral preparation, for example, intravascular administration (preferably, intravenous administration), intraperitoneal administration, intestinal administration, and local administration into a tumor or a vicinity of the tumor can be recited. In one preferred embodiment, the pharmaceutical composition according to the present invention is administered to a subject by intravenous administration or local administration into a tumor or the vicinity of the tumor.

The pharmaceutical composition according to the present invention is used for treating a glioma, preferably a highly invasive glioma, more preferably a high grade glioma. The pharmaceutical composition according to the present invention may be used in combination with other pharmaceuticals or pharmaceutical composition for treating a glioma.

The "treatment" used in this specification covers every type of medically acceptable prophylactic and/or therapeutic intervention intended, for example, for cure, transient remission or prevention of a disease. That is, the treatment of a glioma covers medically acceptable intervention intended for various purposes, including retardation or stop of the progression of a glioma, regression or disappearance of a lesion, prevention of development or prevention of recurrence.

Further, it is expected that a glioma can be treated by administering a substance that suppresses expression and/or function of ACTC1 protein or a pharmaceutical composition containing the substance to a glioma patient. Thus, the present disclosure also provides a method for treating a glioma by administering an effective amount of a substance that suppresses the expression and/or function of ACTC1 protein or a pharmaceutical composition containing the substance for a glioma patient. Here, the "effective amount" means an amount that is effective for treating a glioma, and the amount is appropriately adjusted depending on the malignancy of a glioma, the contents of treatment, the patient and other medical factors.

The present disclosure further provides use of the present biomarker for determining presence or absence of a glioma in a sample. Since it is known that the expression of ACTC1 is little observed in normal glia cells, the present biomarker is useful for detecting a glioma.

The present disclosure further provides a diagnostic imaging method of a glioma for examining the presence or absence, position, extension of a lesion and the like of a glioma on an image by administering a compound capable of specifically binding with ACTC1, the compound being labeled with a substance that emits a signal detectable from outside a living body, for example, an anti-ACTC1 antibody labeled with a fluorescent substance, and detecting the signal.

The present invention will be described more specifically by the following Examples.

Examples

1. Materials and methods

(1-1) Patients and tissue

The clinical study was conducted under the approval of the Institutional Review Board of Sapporo Medical University. Patients between 2 and 84 years of age with diagnosed WHO Grade I to IV gliomas at Sapporo Medical University hospital were eligible for this study, and a letter of consent was obtained from each patient according to the ethical guideline of the facility. All tumors were maximally resected to preserve neurological function and followed with radiochemotherapy in the case of high grade gliomas. Glioma tissues obtained in the operating room between October 2006 and October 2014 were fixed in liquid buffered formalin, and a total of 50 formalin-fixed, paraffin-embedded (FFPE) tissue samples, including 4 WHO Grade I samples, 13 WHO Grade II samples, 7 WHO Grade III samples, and 26 WHO Grade IV samples, were analyzed for expression of ACTC1 with the following procedure.

(1-2) Analysis of expression of ACTC1

A. Analysis of gene expression

A FFPE sample was sliced into thin sections using a microtome and collected in 2-mL microtubes. RNA extraction was preformed using Deparaffinization Solution (QIAGEN) and RNeasy FFPE Kit (QIAGEN) according to the manufacturer's protocol. The total RNA concentration and A260/A280 ratio were measured using a NanoDrop Lite spectrophotometer (Thermo Fisher Scientific Inc.). Samples in which the A260/A280 ratio was less than 1.8 were excluded. Samples with an RNA concentration of less than 40 ng/$\mu$L were prepared using a centrifugal concentrator. The QuantiTect Reverse Transcription Kit (Qiagen) was used to reverse transcribe 500 ng of total RNA. PCR reactions which were 10 $\mu$L in

volume, were prepared with 2 $\mu$L cDNA diluted to 1:5. TaqMan Universal Master Mix II with UNG and TaqMan Gene Expression Assays for glyceraldehyde 3-phosphate dehydrogenase (GAPDH;Hs02758991_g1) and ACTC1 (Hs00606316_m1) were purchased from Thermo Fisher Scientific Inc.. qRT-PCR was performed in double using PRISM7500 (Thermo Fisher Scientific Inc.). PCR conditions were 50°C for 2 minutes, 95°C for 10 minutes, followed by 60 cycles of 95°C for 15 seconds and 60°C for 1 minute. The expression level was quantified by a comparative Ct method. To be more specific, the Ct value of the target gene was compared with the Ct value of the intrinsic control GAPDH to calculate a $\Delta$Ct value, and then fold change (FC) of each sample was determined as a relative value to the calibrator by using the formula 2^(-$\Delta\Delta$Ct). As the calibrator, a numerical value obtained by averaging $\Delta$Ct values in a pooled normal mRNA in which mRNAs extracted from normal brain tissues (n = 5) that were not affected with a glioma were pooled was used. The following is a formula for calculating FC.

[Formula 1]

$$\Delta Ct_{(ACTC1)} = Ct_{(ACTC1)} - Ct_{(GAPDH)}$$

$$\Delta Ct_{(Normal)} = Ct_{(Normal)} - Ct_{(GAPDH)}$$

$$\Delta\Delta Ct_{(ACTC1)} = \Delta Ct_{(ACTC1)} - \Delta Ct_{(Normal)}$$

$$FC\ (fold\ change) = 2^{-\Delta\Delta Ct}$$

When FC calculated by the above formula was less than 1.0 which is the cutoff value, expression of ACTC1-mRNA was determined as negative, whereas when FC was more than or equal to 1.0, expression of ACTC1-mRNA was determined as positive.

B. Analysis of protein expression

A FFPE sample of one case of glioblastoma was sliced into thin sections, and deparaffinized, and then subjected to immune tissue staining using an anti-ATCT1 antibody (alpha Cardiac Muscle Actin antibody, GeneTex) as a primary antibody, and a fluorescent antibody (goat anti-rabbit IgG, Alexa Fluor 488 conjugate, Thermo Fisher Scientific) as a secondary antibody. After staining cell nuclei with DAPI, the cells were observed under a confocal microscope. Also for U87 which is a glioblastoma cell strain purchased from ATCC, immunostaining was conducted in the same manner to analyze expression of ATCT1 protein.

(1-3) Surveillance and follow-up of patients

Overall survival (OS) and progression free survival (PFS) of patients were analyzed using the Kaplan-Meier survival analysis. Tumor progression was defined by tumor size, new areas of tumor, or increase in unequivocal neurological deterioration. Every patient was followed up for up to 15.4 years.

(1-4) Measurement of MIB-1 index

A FFPE block was sliced into thin sections on slides, and deparaffinized. Sections were immunostained with an anti-Ki67 monoclonal antibody using BGX-Ki67 (BioGenex) according to the manufacturer's instructions. The MIB-1 index was calculated as the percentage of positively stained tumor cell nuclei in the area that was highly immunostained.

(1-5) Knockdown of ACTC1

A siRNA (Stealth siRNAs, Thermo Fisher Scientific) that is designed to suppress expression of ACTC1-mRNA was introduced into glioblastoma cell strain U87 using Lipofectamine (registered trademark) RNAiMAX Transfection Reagent (Thermo Fisher Scientific), and ACTC1-mRNA was measured by the same method as (1-2) A., to investigate suppression of expression by the siRNA.

(1-6) Migration assay

For evaluating the migration ability of U87 in which expression of ACTC1-mRNA was suppressed by the siRNA, a migration assay was performed using CytoSelect (registered trademark) 24-Well Cell Migration Assay Kit (CELL BIOLABS, INC.) according to the manufacturer's instruction. In each well of the assay plate into which a culture medium was dispensed, a chamber having a bottom of polycarbonate membrane was installed, and a cell suspension was put into the chamber, and stood still for 6 hours to allow migration of the cells. The migrating cells having passed the membrane were stained with a cell staining solution, and absorbance at a wavelength of 560 nm was measured.

(1-7) Statistical analysis

Differences among groups were assessed using the Kruskal-Wallis test, and the Mann-Whitney U test and the Spearman test. All statistical analyses were performed using SPSS (version 22) (International Business Machines Corporation). Differences were deemed statistically significant if $p < 0.05$. Comparison of survival was performed with the log-rank test.

2. Results

(2-1) Analysis of expression of ACTC1

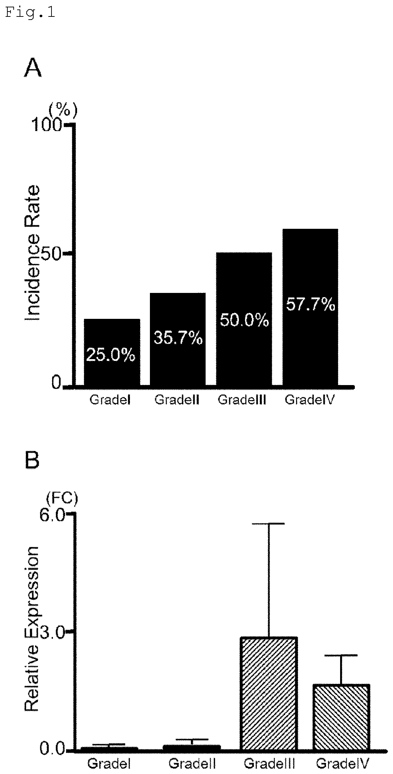

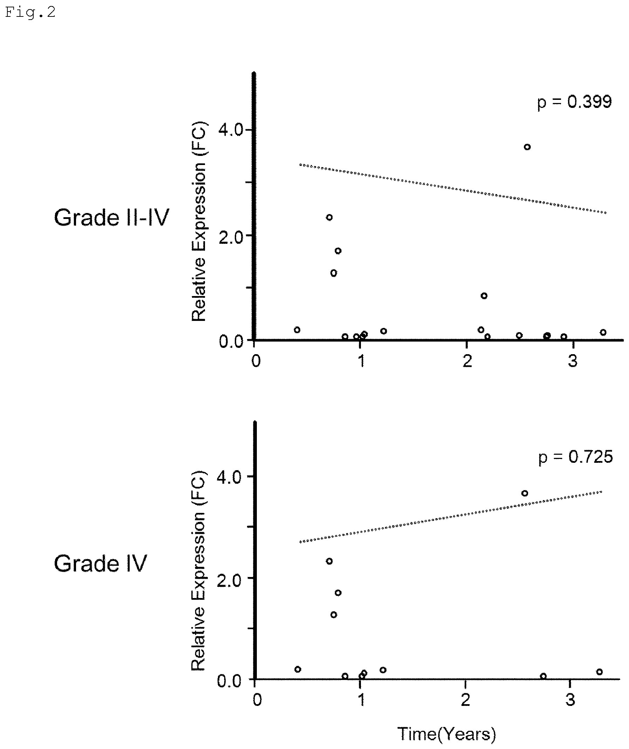

Results of the analysis of gene expression are shown in Fig. 1 and Fig. 2. The ACTC1-mRNA expression rate as a qualitative assessment increased as the WHO Grade elevated (Fig. 1A). The ACTC1-mRNA expression level as a quantitative assessment for the cases in which ACTC1-mRNA was detected significantly increased in high grade gliomas (Grade III and IV) as compared with low grade gliomas (Grade I and II) (Fig. 1B, $p = 0.024$). Further, the relationship between the ACTC1-mRNA expression level and the prognosis were assessed for the cases in which ACTC1-mRNA was detected (Fig. 2). Since no correlation was observed between the ACTC-mRNA expression level and the overall survival (OS) in any of gliomas excluding Grade I (Grade II to IV) and gliomas of Grade IV, it is considered that presence or absence of expression of ACTC1 rather than the expression level of ACTC1 is correlated with the prognosis.

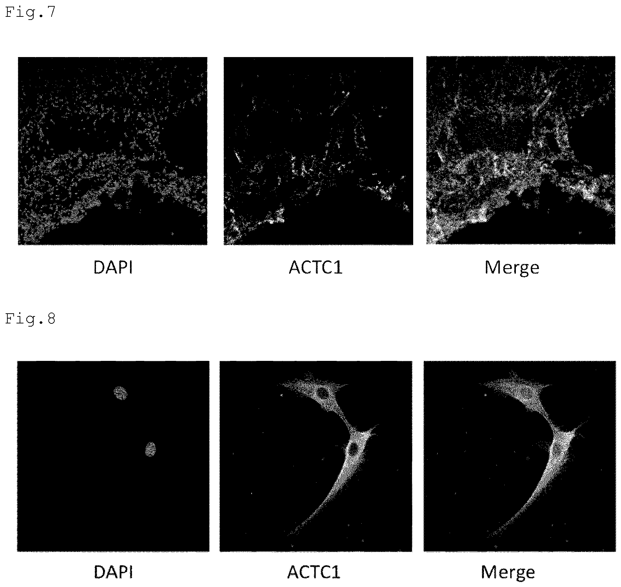

Results of analysis of protein expression are shown in Fig. 7 and Fig. 8. In any of glioblastoma tissue, and glioblastoma cell strain, expression of ACTC1 protein was observed.

(2-2) Analysis of overall survival (OS) and progression free survival (PFS) by Kaplan-Meier method

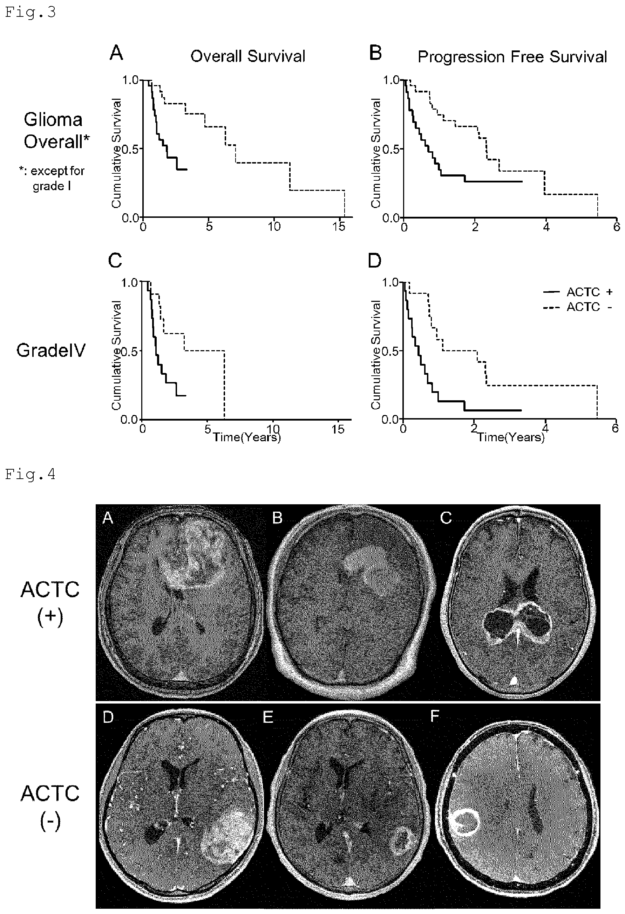

Fig. 3 shows Kaplan-Meier curves of the overall survival (OS) and progression free survival (PFS) of the patients, and Table 1 shows the result of analysis of the hazard ratio for the medians by fitting to the Cox proportional hazard models.

[Table 1]

| | | ACTC1 negative | ACTC1 positive | Benefit by ACTC1 negativity | p value | Hazard ratio | 95% confidence interval |
|---|---|---|---|---|---|---|---|
| Median Overall Survival (mOS) (year) | Glioma (except for grade I) | 6.28 (n=23) | 1.84 (n=23) | 4.44 | 0.006 | 4.40 | 1.53-12.63 |
| | GradeIV | 3.20 (n=11) | 1.08 (n=15) | 2.12 | 0.044 | 3.01 | 1.03-8.79 |
| Median Progression Free Survival (mPFS) (year) | Glioma (except for grade I ) | 2.34 (n=23) | 0.70 (n=23) | 1.64 | 0.040 | 2.16 | 1.04-4.46 |
| | GradeIV | 1.13 (n=11) | 0.44 (n=15) | 0.69 | 0.020 | 2.92 | 1.19-7.16 |

In any of gliomas excluding Grade I and glioblastomas, the mOS and mPFS of the ACTC1-positive group were with statistical significance shorter than those of the ACTC1-negative group. This indicates that ACTC1 is an unfavorable prognostic factor for glioma.

In the present study, the mOS of glioblastoma that was subjected to radiochemotherapy with temozolomide after maximally resecting tumors was 18.4 months (1.53 years) which is comparable with that recently reported. Further, the survival benefit by IDH mutation that is known as a known favorable prognostic factor has been reported as 16 months (1.33 years) (Yan H et al. (2009), N. Engl. J. Med. 360(8): 765-773). The survival benefit by ACTC1 negativity in this study was demonstrated to be longer than the survival benefit by such chemotherapy or IDH mutation.

(2-3) MRI findings at the time of diagnosis

At the time of diagnosis, invasion toward the contralateral cerebral hemisphere was observed in 31.6% of ACTC1-positive glioblastomas, while invasion was not observed in ACTC1-negative glioblastomas (p = 0.020). Typical MRI findings of ACTC1-positive glioblastoma at the time of diagnosis are shown in Figs. 4A to 4C. Deep-seated invasion toward the contralateral cerebral hemisphere was observed, and a typical invasion pattern was progression through the corpus callosum. In both of a 60-year-old female patient presented with symptoms of headache and nausea (Fig. 4A) and a 53-year-old male patient presented with symptoms of seizure (Fig. 4B), the tumor invaded toward the contralateral cerebral hemisphere through the genu and anterior third of the corpus callosum. MRI of a 71-year-old female patient presented with symptoms of headache showed invasion of a glioblastoma through the splenium of the corpus callosum (Fig. 4C).

Typical MRI findings of ACTC-negative glioblastoma at the time of diagnosis are shown in Figs. 4D to 4F. Tumors developed near the surface of the cortex and were topographically uniform. In a 19-year-old female patient presented with symptoms of progressive sensory aphasia, even though the tumor had a maximum diameter of 62 mm, invasion was not observed (Fig. 4D). The similar findings were observed in the tumors of a 63-year-old female patient presented with symptoms of sensory aphasia (Fig. 4E), and a 61-year-old woman (Fig. 4F) presented with symptoms of left hemiparesis.

(2-4) MRI findings at the time of recurrence of glioblastomas

At the time of recurrence, in high grade gliomas, remote recurrence was observed in 90.9% of ACTC1-positive cases, while ACTC-negative cases did not demonstrate remote recurrence (p = 0.000). In the case of gliomas of Grade III, recurrence was observed in 71.4% of cases, and all the ACTC1-positive cases demonstrated remote recurrence, while recurrence occurred only in localized areas in all the ACTC1-negative cases (p = 0.025). In the case of glioblastomas, 87.5% of ACTC1-positive cases demonstrated remote recurrence, while remote recurrence was not observed in ACTC1-negative cases (p = 0.007).

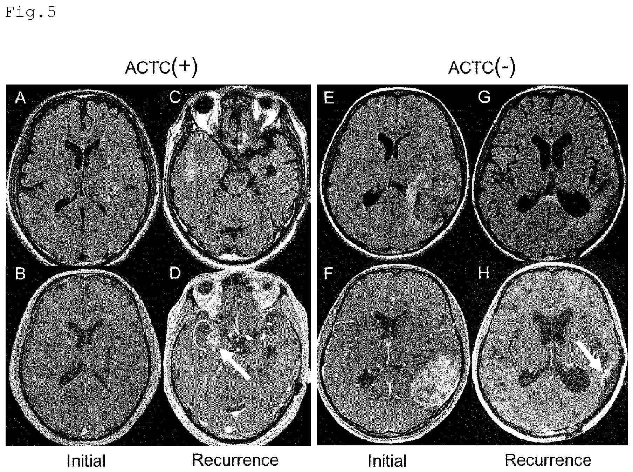

Typical MRI findings of ACTC-positive glioblastoma at the time of recurrence are shown on the left side of Fig. 5. The tumor of a 57-year-old male patient presented with symptoms of left sensory disturbance at the time of diagnosis originated from the left putamen and exhibited heterogeneous ring enhancement with perifocal edema (Figs. 5A and 5B). Recurrence in this case was observed in the right temporal lobe at 20.3 months after initial surgery (Figs. 5C and 5D). The recurrent lesion was in the contralateral side with presumed tumor spread through the commissural tracts.

Typical MRI findings of ACTC-negative glioblastoma at the time of recurrence are shown on the right side of Fig. 5. The tumor of a 19-year-old female patient presented with symptoms of progressive sensory aphasia at the time of diagnosis was localized at the posterior temporal lobe with perifocal edema (Figs. 5E and 5F). In this case, since the tumor was confined to a superficial location, gross-total removal of the enhanced tumor was accomplished. At 13.3 months after initial treatment, only local recurrence without any invasion toward multiple lobules or remote recurrence was observed (Figs. 5G and 5H).

(2-5) Relationship among ACTC1 expression and patient age, Karnofsky performance status (KPS) and MIB-1 index

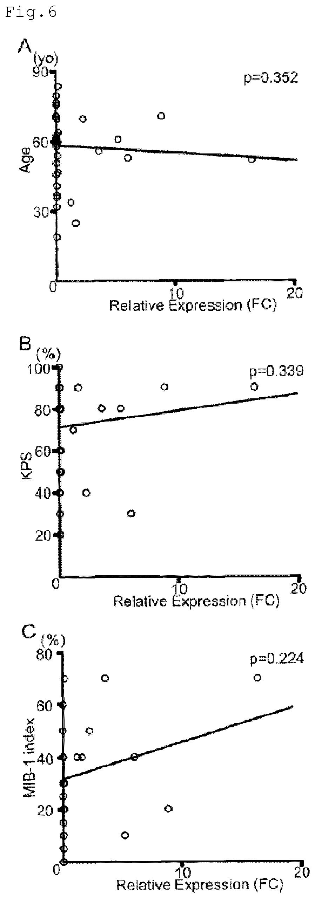

For all glioma cases of Grade I to IV, relationships between ACTC1 expression, and each of patient age and KPS which are known as prognostic factors for glioma, and MIB-1 index which is an index of cell proliferating ability were assessed. Results are shown in Figs. 6. No correlation was observed between ACTC1-mRNA expression level and patient age (Fig. 6A), KPS (Fig. 6B) and MIB-1 index (Fig. 6C).

(2-6) Assessment of influence of suppression of ACTC1-mRNA expression on cell migration ability

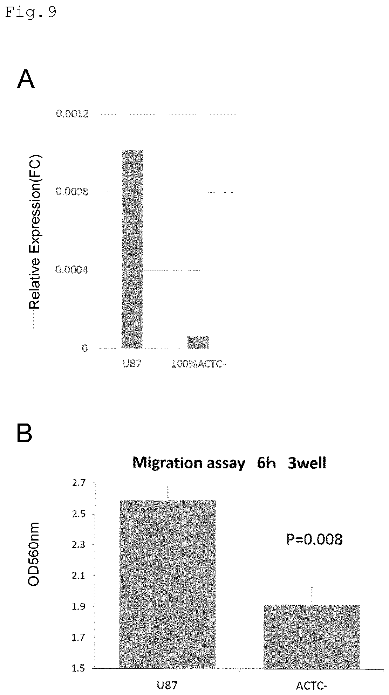

The influence of suppression of the expression of ACTC1-mRNA on cell migration ability was assessed by using glioblastoma cell strain U87. The siRNA significantly suppressed expression of ACTC1-mRNA in U87 (Fig. 9A). The result of the migration assay revealed that the migration ability was greatly deteriorated in U87 in which expression of ACTC1-mRNA was suppressed by the siRNA treatment, as compared with the migration ability of U87 that was not subjected to the siRNA treatment (Fig. 9B).

The results of (2-1) to (2-5) revealed that ACTC1 is a glioma prognostic factor that is independent of patient age and KPS. Further, since no correlation was observed between ACTC1 and MIB-1 index, it was estimated that the poor prognosis of ACTC1-positive glioblastoma is due to tumor cell invasion along the nerve tracts, not cell proliferation. ACTC1 is considered as an invasion marker that is independent of proliferation.

The result of (2-6) revealed that by suppressing the expression of ACTC1, migration of glioma cells is inhibited, namely invasion and remote recurrence of glioma can be suppressed.

**Claims**

1. A method for determining the prognosis of a glioma, comprising: a step of detecting actin alpha cardiac muscle 1 (ACTC1) protein and/or mRNA encoding ACTC1 protein in a sample containing glioma collected from a patient, and a step of determining that the glioma has a poor prognosis when ACTC1 protein and/or mRNA encoding ACTC1 protein is detected in the detecting step.

2. A method for determining the risk of remote recurrence of a glioma, comprising: a step of detecting ACTC1 protein and/or mRNA encoding ACTC1 protein in a sample containing a glioma collected from a patient, and a step of determining that the glioma has a high risk of remote recurrence when ACTC1 protein and/or mRNA encoding ACTC1 protein is detected in the detecting step.

3. A method for determining the invasion ability of a glioma cell, comprising: a step of detecting ACTC1 protein and/or mRNA encoding ACTC1 protein in a sample containing a glioma cell, and a step of determining that the glioma cell has a high invasion ability when ACTC1 protein and/or mRNA encoding ACTC1 protein is detected in the detecting step.

4. A method for determining the presence of a highly invasive glioma in a sample collected from a glioma lesion or the vicinity of the glioma lesion in a patient, comprising a step of detecting ACTC1 protein and/or mRNA encoding ACTC1 protein in the sample, and a step of determining that the sample contains highly invasive glioma when ACTC1 protein and/or mRNA encoding ACTC1 protein is detected in the detecting step.

5. Use of a kit in a method according to claim 1 or 2 for determining the prognosis of a glioma and/or the risk of remote recurrence of glioma, comprising a means that detects ACTC1 protein and/or mRNA encoding ACTC1 protein in a sample containing a glioma collected from a patient.

6. Use of a kit in a method according to claim 3 for determining the invasion ability of a glioma cell, comprising a means that detects ACTC1 protein and/or mRNA encoding ACTC1 protein in a sample containing a glioma cell.

7. Use of a kit in a method according to claim 4 for determining the presence of a highly invasive glioma in a sample collected from a glioma lesion or the vicinity of the glioma lesion in a patient, comprising a means that detects ACTC1 protein and/or mRNA encoding ACTC1 protein in the sample.

8. Use of a kit according to any one of Claims 5 to 7, comprising at least one of a specific antibody for detecting ACTC1 protein or a primer nucleic acid or a probe nucleic acid for detecting mRNA encoding ACTC1.

9. A pharmaceutical composition comprising a substance that suppresses the expression and/or function of ACTC1 protein for use in treating glioma, wherein the substance that suppresses expression and/or function of ACTC1 protein is selected from the group consisting of an inhibitory nucleic acid against a gene encoding ACTC1 protein, a recombinant virus capable of expressing an inhibitory nucleic acid against a gene encoding ACTC1 protein, and a neutralizing antibody against ACTC1 protein.

**Patentansprüche**

1. Verfahren zum Bestimmen der Prognose eines Glioms, umfassend: einen Schritt des Nachweisens von alpha-Herzmuskel-Aktin-1(ACTC1)-Protein und/oder von für ACTC1-Protein kodierender mRNA in einer einem Patienten entnommenen Probe, die Gliom enthält, und einen Schritt des Nachweisens, dass das Gliom eine schlechte Prognose hat, wenn das ACTC1-Protein und/oder die für ACTC1-Protein kodierende mRNA in dem Nachweisschritt nachgewiesen wird.

2. Verfahren zum Bestimmen des Risikos eines Fernrezidivs eines Glioms, umfassend: einen Schritt des Nachweisens von ACTC1-Protein und/oder für ACTC1-Protein kodierender mRNA in einer einem Patienten entnommenen Probe, die Gliom enthält, und einen Schritt des Bestimmens, dass das Gliom ein hohes Risiko eines Fernrezidivs hat, wenn ACTC1-Protein und/oder für ACTC1-Protein kodierende mRNA in dem Nachweisschritt nachgewiesen wird.

3. Verfahren zum Bestimmen der Invasionsfähigkeit einer Gliomzelle, umfassend: einen Schritt des Nachweisens von ACTC1-Protein und/oder für ACTC1-Protein kodierender mRNA in einer Probe, die eine Gliomzelle enthält, und einen Schritt des Bestimmens, dass die Gliomzelle eine hohe Invasionsfähigkeit hat, wenn ACTC1-Protein und/oder für ACTC1-Protein kodierende mRNA in dem Nachweisschritt nachgewiesen wird.

4. Verfahren zum Bestimmen des Vorhandenseins eines hochinvasiven Glioms in einer Probe, die von einer Gliomläsion oder der Umgebung der Gliomläsion in einem Patienten entnommen wurde, umfassend einen Schritt des Nachweisens von ACTC1-Protein und/oder für ACTC1-Protein kodierender mRNA und einen Schritt des Bestimmens, dass die Probe ein hochinvasives Gliom enthält, wenn ACTC1-Protein und/oder für ACTC1-Protein kodierende mRNA in dem Nachweisschritt nachgewiesen wird.

5. Verwendung eines Kits in einem Verfahren nach Anspruch 1 oder 2 zum Bestimmen der Prognose eines Glioms und/oder des Risikos eines Fernrezidivs eines Glioms, umfassend ein Mittel, das ACTC1-Protein und/oder für ACTC1-Protein kodierende mRNA in einer von einem Patienten entnommenen Probe, die ein Gliom enthält, nachweist.

6. Verwendung eines Kits in einem Verfahren nach Anspruch 3 zum Bestimmen der Invasionsfähigkeit einer Gliomzelle, umfassend ein Mittel, das ACTC1-Protein und/oder für ACTC1-Protein kodierende mRNA in einer Probe nachweist, die eine Gliomzelle enthält.

7. Verwendung eines Kits in einem Verfahren nach Anspruch 4 zum Bestimmen des Vorhandenseins eines hochinvasiven Glioms in einer Probe, die aus einer Gliomläsion oder der Umgebung der Gliomläsion in einem Patienten entnommen wurde, umfassend ein Mittel, das ACTC1-Protein und/oder für ACTC1-Protein kodierende mRNA in der Probe nachweist.

8. Verwendung eines Kits nach einem der Ansprüche 5 bis 7, umfassend mindestens einen spezifischen Antikörper zum Nachweisen des ACTC1-Proteins oder eine Primer-Nukleinsäure oder eine Sonden-Nukleinsäure zum Nachweisen von für ACTC1 kodierender mRNA.

9. Pharmazeutische Zusammensetzung, umfassend eine Substanz, die die Expression und/oder Funktion des ACTC1-Proteins unterdrückt, zur Verwendung bei der Behandlung eines Glioms, wobei die Substanz, die die Expression und/oder Funktion des ACTC1-Proteins unterdrückt, aus der Gruppe bestehend aus einer inhibitorischen Nukleinsäure gegen ein für ACTC1-Protein kodierendes Gen, einem rekombinanten Virus, der eine inhibitorische Nukleinsäure gegen ein für ACTC1-Protein kodierendes Gen exprimieren kann, und einem neutralisierenden Antikörper gegen das ACTC1-Protein ausgewählt ist.

**Revendications**

1. Procédé pour déterminer le pronostic d'un gliome, comprenant : une étape consistant à détecter la protéine actine alpha du muscle cardiaque 1 (ACTC1) et/ou un ARNm codant la protéine ACTC1 dans un échantillon contenant un gliome prélevé sur un patient, et une étape consistant à déterminer que le gliome a un pronostic défavorable lorsque la protéine ACTC1 et/ou un ARNm codant la protéine ACTC1 est détecté(e) dans l'étape de détection.

2. Procédé pour déterminer le risque de récidive éloignée d'un gliome, comprenant : une étape consistant à détecter la protéine ACTC1 et/ou un ARNm codant la protéine ACTC1 dans un échantillon contenant un gliome prélevé sur un patient, et une étape consistant à déterminer que le gliome a un haut risque de récidive éloignée lorsque la protéine ACTC1 et/ou un ARNm codant la protéine ACTC1 est détecté(e) dans l'étape de détection.

3. Procédé pour déterminer l'aptitude invasive d'une cellule de gliome, comprenant : une étape consistant à détecter la protéine ACTC1 et/ou un ARNm codant la protéine ACTC1 dans un échantillon contenant une cellule de gliome, et une étape consistant à déterminer que la cellule de gliome a une haute aptitude invasive lorsque la protéine ACTC1 et/ou un ARNm codant la protéine ACTC1 est détecté(e) dans l'étape de détection.

4. Procédé pour déterminer la présence d'un gliome hautement invasif dans un échantillon prélevé à partir d'une lésion gliomateuse ou du voisinage de la lésion gliomateuse chez un patient, comprenant une étape consistant à détecter la protéine ACTC1 et/ou un ARNm codant la protéine ACTC1 dans l'échantillon, et une étape consistant à déterminer que l'échantillon contient un gliome hautement invasif lorsque la protéine ACTC1 et/ou un ARNm codant la protéine ACTC1 est détecté(e) dans l'étape de détection.

5. Utilisation d'un nécessaire dans un procédé selon la revendication 1 ou 2 pour déterminer le pronostic d'un gliome et/ou le risque de récidive éloignée d'un gliome, comprenant un moyen qui détecte la protéine ACTC1 et/ou un ARNm codant la protéine ACTC1 dans un échantillon contenant un gliome prélevé sur un patient.

6. Utilisation d'un nécessaire dans un procédé selon la revendication 3 pour déterminer l'aptitude invasive d'une cellule de gliome, comprenant un moyen qui détecte la protéine ACTC1 et/ou un ARNm codant la protéine ACTC1 dans un échantillon contenant une cellule de gliome.

7. Utilisation d'un nécessaire dans un procédé selon la revendication 4 pour déterminer la présence d'un gliome hautement invasif dans un échantillon prélevé à partir d'une lésion gliomateuse ou du voisinage de la lésion gliomateuse chez un patient, comprenant un moyen qui détecte la protéine ACTC1 et/ou un ARNm codant la protéine ACTC1 dans l'échantillon.

8. Utilisation d'un nécessaire selon l'une quelconque des revendications 5 à 7, comprenant au moins un élément parmi un anticorps spécifique pour la détection de la protéine ACTC1 ou un acide nucléique amorce ou un acide nucléique sonde pour la détection d'ARNm codant ACTC1.

9. Composition pharmaceutique comprenant une substance qui supprime l'expression et/ou la fonction de la protéine ACTC1, destinée à être utilisée dans le traitement d'un gliome, dans laquelle la substance qui supprime l'expression et/ou la fonction de la protéine ACTC1 est choisie dans le groupe constitué par un acide nucléique inhibiteur contre un gène codant la protéine ACTC1, un virus recombinant capable d'exprimer un acide nucléique inhibiteur contre un gène codant la protéine ACTC1, et un anticorps neutralisant contre la protéine ACTC1.

Fig.1

A

B

Fig.2

Grade II-IV

Grade IV

Fig.3

Fig.4

Fig.5

ACTC(+)

ACTC(-)

| A | C | E | G |
| B | D | F | H |

Initial          Recurrence          Initial          Recurrence

Fig.6

Fig.7

DAPI   ACTC1   Merge

Fig.8

DAPI   ACTC1   Merge

Fig.9

**A**

**B**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008054786 A

**Non-patent literature cited in the description**

- **VIGNESWARAN K et al.** *Ann. Transl. Med.,* 2015, vol. 3 (7), 95
- **YAN H et al.** *N. Engl. J. Med.,* 2009, vol. 360 (8), 765-773